(19) 

Europäisches
Patentamt
European
Patent Office
Office européen
des brevets

(11) **EP 1 767 145 A1**

(12) **EUROPEAN PATENT APPLICATION**

(43) Date of publication:
**28.03.2007 Bulletin 2007/13**

(51) Int Cl.:
*A61B 5/029* *(2006.01)*

(21) Application number: **05108898.7**

(22) Date of filing: **27.09.2005**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(71) Applicant: **Pulsion Medical Systems AG
81829 München (DE)**

(72) Inventors:
 • **Bohn, Matthias
  81477 München (DE)**

 • **Goedje, Oliver
  82031 Grünwald (DE)**
 • **Thalmeier, Thomas
  81541 München (DE)**

(74) Representative: **Kehl, Günther
  Kehl & Ettmayr Patentanwälte,
  Friedrich-Herschel-Strasse 9
  81679 München (DE)**

(54) **Apparatus, computer system and computer program for determining cardio-vascular parameters**

(57)    Disclosed is an apparatus, a computer system (104), a computer program, a temperature sensor device (117) and a heat transfer device (107) with which thermodilution measurements can be carried out in a non-invasive or less invasive manner by using extravascular (transcutaneous or subcutaneous) temperature sensor devices and/or extravascular heat transfer devices.

*Fig.1*

EP 1 767 145 A1

## Description

FIELD OF THE INVENTION

The present invention relates to an apparatus, a computer system a computer program, an extravascular temperature sensor device and an extravascular heat transfer device for determining parameters of a patient by thermodilution measurements.

BACKGROUND OF THE INVENTION

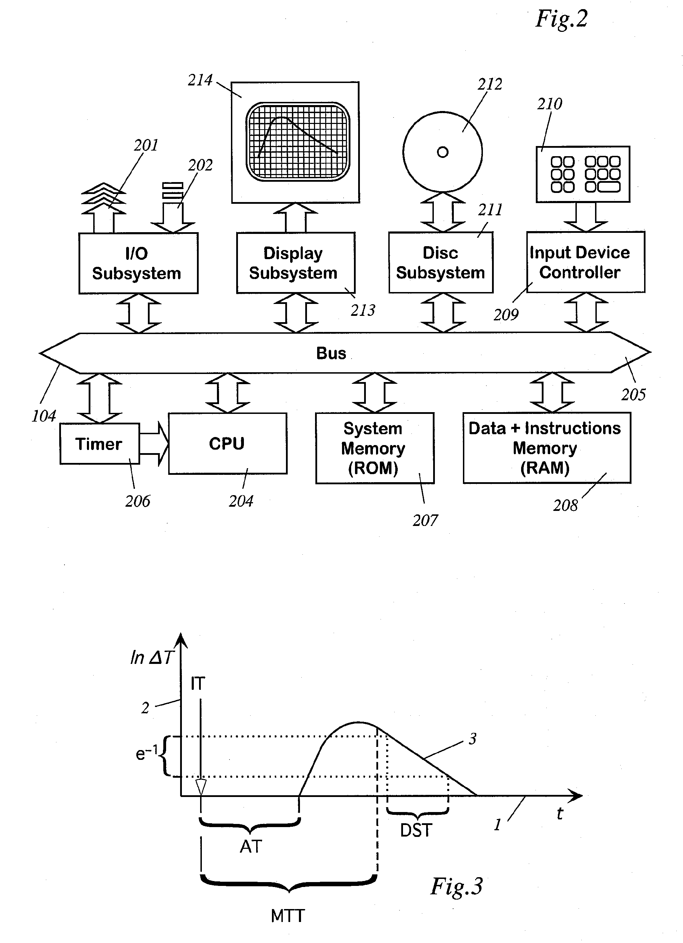

The current state of the art in implementing transpulmonary thermodilution measurement are apparatus for injecting a bolus of thermal indicator into a patient's vena cava superior, and measuring the temperature response at a place of the patient's systemic circulation, e.g. patient's femoral artery to determine the thermodilution curve, i.e. the temperature response as a function of time. From the thermodilution curve, a schematic example of which is illustrated in fig. 3, wherein the abscissa (time axis) 1 is linear and the ordinate (temperature difference axis) 2 is logarithmic, various cardio-vascular parameters can be derived by using computer systems running computer programs, which implement parameter calculations as disclosed in WO 93/21823, the contents of which are included herein by citation, and as set forth briefly below.

The Cardiac Output CO can be determined by algorithms based on the Stewart-Hamilton-equation:

$$CO = \frac{V_L(T_B - T_L)K_1 K_2}{\int \Delta T_B(t)dt}$$

where $T_B$ is the initial blood temperature, $T_L$ is the temperature of the liquid bolus, which is used as thermal indicator, VL is the thermal indicator volume, $K_1$ and $K_2$ are constants to consider the specific measurement set-up, and $\Delta T_B(t)$ is the blood temperature as a function of time with respect to the baseline blood temperature $T_B$. Thermal indicator can either be colder or warmer with respect to blood temperature. To obtain cardiac output, the area under the thermodilution curve has to be determined by mathematical integration.

Other parameters that can be derived from the thermodilution curve 3 as schematically illustrated in fig. 3 include the Exponential Decay or Downslope Time DST, i.e. the time the blood temperature difference $\Delta T_B$(t) takes to drop by the factor 1/e, the Appearance Time AT, i.e. the time span between bolus injection IT and first appearance of a noticable temperature difference $\Delta T_B$(t) and the Mean Transit Time MTT.

Transpulmonary thermodilution has been shown to be a reliable technique for assessing cardiac output (CO), cardiac preload and extravascular lung water (EVLW), i.e. to quantify pulmonary edema.

Measuring the temperature response at a place of the patient's systemic circulation, e.g. patient's femoral artery, to determine the thermodilution curve, i.e. the temperature response as a function of time, has been made in the prior art by an invasive measurement. This means that a catheter must be advanced into the femoral artery which is - due to the blood pressure inside the artery - a time consuming and cumbersome procedure which is not without risk for the patient.

US 2003/0130587 A1 discloses a non-invasive method of cardiac output measurement through assessment of a skin thermal response. By warming of a previously cooled digital thermometer placed on patient's wrist the blood flow velocity within the arteria radialis can be calculated. However, the blood flow velocity in the arteria radialis allows only a very rough approximation of the cardiac output. Particularly, any anomalies in the arteria radialis would result in a completely wrong approximation of cardiac output.

SUMMARY OF THE INVENTION

It is therefore an object of the present invention to provide a new apparatus, a new computer system and a new computer program a new extravascular temperature sensor device and a new extravascular heat transfer device allowing the determination of cardiovascular parameters such as cardiac output by transpulmonary thermodilution which allows a patient-friendly, quick and riskless determination of the arterial thermodilution curve and still allows the determination of cardiac output and other parameters with an adequate accuracy and reliability at least for a preliminary estimate.

It has been found that using a non-invasive thermometer, which may be pressed on patient's skin over the, for example, arteria radialis or the arteria femoralis or any other artery which is close to the skin, such as the arteria carotis, provides the thermodilution curve with adequate accuracy so that the cardiac output can be reliably determined from the thermodilution curve according to the Stewart-Hamilton-equation as explained above.

In order to accomplish the above mentioned object, the invention provides an apparatus for determining at least one cardio-vascular parameter of a patient by thermodilution measurements comprising: temperature influencing means for provoking an initial local temperature change in the proximity of a first place of a patient's vascular system thus introducing a travelling temperature deviation to patient's blood stream, an extravascular temperature sensor device for measuring the local temperature of patient's blood at a second place of patient's vascular system downstream of said first place and a computer system coupled to said temperature sensor device for recording said patient's local blood temperature measured at said second place as a function of time to determine a thermodilution curve.

In order to accomplish the above mentioned object, the invention provides also a computer system comprising first coupling means to couple said computer system to temperature influencing means and second coupling means to connect said computer system to an extravascular temperature sensor device and accessing means to acces executable instructions to cause said computer system to control temperature influencing means connected to said computer system to provoke an initial local temperature change in the proximity of a first place of a patient's vascular system, thus introducing a travelling temperature deviation to patient's blood stream, to record said patient's local blood temperature measured by a temperature sensor device at a second place of patient's vascular system downstream of said first place as a function of time to determine a thermodilution curve.

In order to accomplish the above mentioned object, the invention further provides a computer program and a storage medium having physically stored thereon the computer program for determining a cardio-vascular parameter of a patient by thermodilution measurements comprising instructions executable by a computer system to cause said computer system to control temperature influencing means coupled to said computer system to provoke an initial local temperature change in the proximity of a first place of a patient's vascular system, thus introducing a travelling temperature deviation to patient's blood stream, and to record said patient's local blood temperature measured by an extravascular temperature sensor device at a second place of patient's vascular system downstream of said first place as a function of time to determine a thermodilution curve.

In order to accomplish the above mentioned object, the invention further provides an extravascular subcutaneous temperature sensor device and an extravascular subcutaneous heat transfer device.

Further advantageous embodiments are described in the subclaims.

The accompanying drawings serve for a better understanding of the above and other features of the present invention.

BRIEF DESCRIPTION OF THE DRAWINGS

Fig.1 shows a schematic sketch of both a patient's vascular system and a preferred embodiment of an apparatus according to the present invention.

Fig.2 shows a block diagram illustrating the general hardware structure of an embodiment of a computer system according to the present invention being part of the apparatus sketched in fig.1.

Fig.3 shows a schematic example of a Thermodilution Curve in a diagram with the blood temperature difference as a function of time, wherein the abscissa is linear and the ordinate is logarithmic.

Fig.4 shows a schematic example of a subcutaneous extravascular temperature sensor device.

Fig.5 shows the subcutaneous extravascualar temperature sensor device after having been pierced through patient's skin.

Fig.6 shows the subcutaneous extravascular temperature sensor device after having been pierced through patient's skin and secured with a self-adhesive strip.

Fig.7 shows a transcutaneous heat transfer device for transcutaneous heat transfer.

Fig.8 shows a subcutaneous extravascular heat transfer device.

Fig.9 shows a schematic sketch of both a patient's vascular system and a preferred embodiment of an apparatus adapted provide data for carrying out a system calibration,

Fig.10 shows a schematic sketch of both a patient's vascular system and a preferred embodiment of an apparatus adapted carry out a system calibration by means of the data provided as shown in Fig. 9.

DETAILED DESCRIPTION

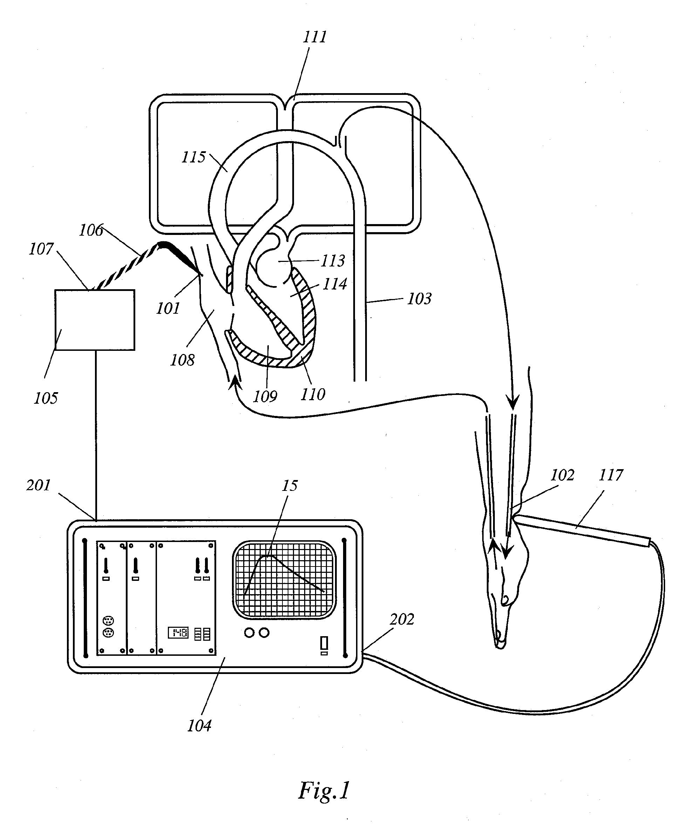

Figure 1 illustrates the main components necessary to implement an embodiment of an apparatus according to the invention and schematically shows the first and second places 101, 102 of a patient's vascular system 103, where the apparatus interacts with the patient's vascular system 103. A computer system 104, the general hardware structure of which is schematically illustrated in figure 2, is connected via port 201 with a medical dosage device 105 serving together with a catheter 106 as an injection means 107 to inject at the first place 101, e.g. into patient's vena cava superior, a bolus, e.g. 10 ml, or, as a guideline, 0.15 ml/kg patient's body mass. The bolus serving as a thermal indicator liquid is substantially warmer or colder than patient's blood temperature. As a result, a travelling temperature deviation is introduced to the patient's vascular system 103, where it continuously changes according to boundary conditions. The travelling temperature deviation passes right atrium and right ventricle 109 of patient's heart 110 to enter the pulmonary circulation 111. The travelling temperature deviation passes the left atrium 113 and the left ventricle 114 of patient's heart to enter through the aorta 115 the systemic circulation. When the travelling temperature deviation reaches the second place 102, for example patient's arteria radialis or any other artery which is close to the skin, such as the arteria femoralis or arteria carotis, where the patient's blood temperature is continuously measured by an extravascular sensor device 117, which is connected to the computer system 104 via port 202, the travelling temperature deviation is recorded by the computer system 104 as Thermodilution Curve 15, i.e. temperature measured at the second place 102 as function of time. From this Thermodilution Curve 15 the computer system 104 calculates the cardiac output according to the Stewart-Hamilton-equation as explained above. Other parameters, such as mean transit time and down-slope time may be calculated if required.

The non-invasive transcutaneous temperature sensor device 117 is pressed against patient's skin to bring it as close as possible to an artery such as the arteria radialis or arteria femoralis or any other artery of similar size which is close to the skin, such as the arteria carotis, for instance. The temperature and in particular the change of the temperature during the course of time is measured whereby use is made of thermal conduction or infrared radiation. In the first case the non-invasive thermometer includes a semiconductor thermoelement, whereas in the second case a bolometer which determines the temperature from infrared radiation may be used.

If the examination shows that the cardiac output is not in a range that can be regarded to be entirely safe, the doctor may arrange for a further CO-determination with a conventional invasive sensor device. The computer program according to a particular advantageous embodiment of the invention provides for this purpose that a warning signal is generated indicating that cardiac output is below a certain threshold and the determination of the cardiac output should be repeated using a conventional invasive temperature sensor device. In this way CO-determination under invasive temperature measurement is restricted to critical cases only, and invasive temperature measurement can be avoided for many patients.

Figure 2 illustrates the general hardware structure of an embodiment of a computer system 104 according to the invention, suitable to be part of the apparatus shown in Fig.1. Via ports 201, 202 which belong to an input/output subsystem, the computer system 104 is connectable to temperature influencing means 107 and an extravascular temperature sensor device 117, respectively. The input/output subsystem is controlled by a central processing unit (CPU) 204, which communicates via a data and adress bus 205 with the other components of the computer system 104, which include a timer 206 providing timer clock signals to the CPU 204, a system memory (ROM) 207, in which the system software is permanently stored, a data and instructions memory (RAM) 208, where both executable instructions and various data including temperature readings for thermodilution curves readings can be stored, an input device controller 209 controlling an input device 210, such as a keypad, a touch screen or the like, for manually entering system parameters, operation settings and the like, a disc subsystem 211 to read data or program instructions from a storage medium 212, such as a hard disc, floppy disc, compact disc, optical disc or the like, and to store data to the storage medium 212, and a display subsystem 213 controlling a display 214 to display relevant information, such as a Thermodilution Curve or cardio-vascular parameters determined by the computer system 104.

The above described apparatus is adapted to determine MTT, DST, CO from the thermodilution curve.

Instead of pressing the transcutaneous sensor device 117 - as shown in figure 1 - against patient's outer skin, a subcutaneous extravascular sensor device may be used as described below.

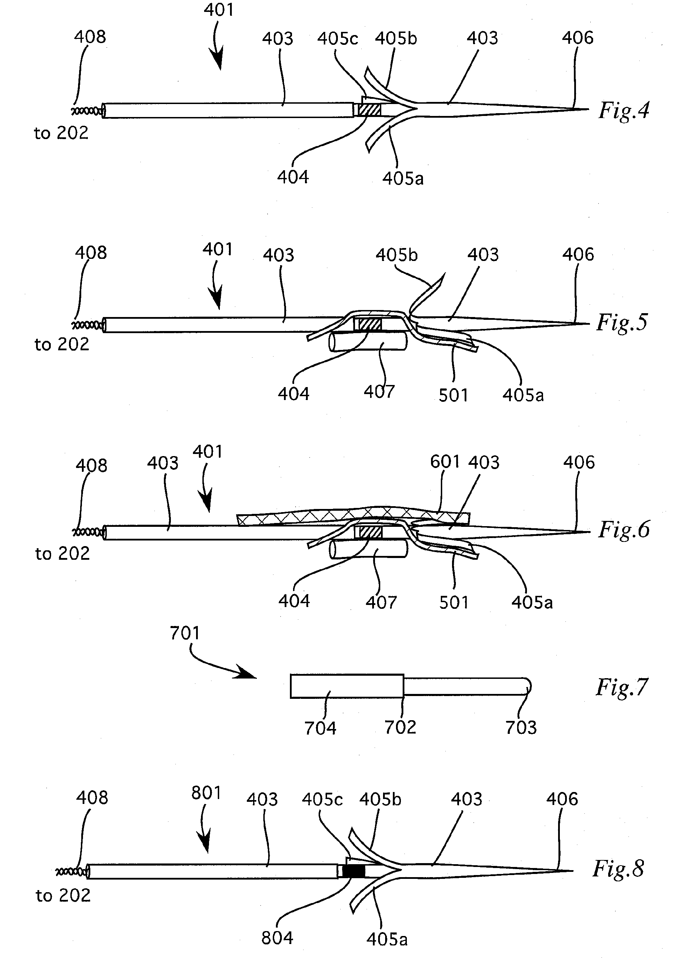

Figure 4 illustrates an example of a subcutaneous extravascular temperature sensor device 401 which comprises a thermal contact portion 404 adapted to be advanced under the patient's skin to get into heat transfer contact with a blood vessel. The temperature sensor device 401 has the overall form of an epidermic needle which is partially covered with a coating 403. The temperature sensor device 401 comprises a thermal contact portion 404. A thermometer, such as a thermistor or a bolometer is located on the temperature sensor device 401 and constitutes the contact portion 404 (hatched area). The coating 403 is provided with several cut lines so as to form flaps 405a, 405b and 405c. The flaps 405 have been folded back from the contact portion 404, so that the contact portion 404 is exposed. The temperature sensor device 401 has a sharp tip 406 which allows to pierce it through patient's skin. The subcutaneous extravascular sensor device 401 is coupled via a pair of wires 408 to the port 202 of computer system 104 of figure 1. The procedure of carrying out the measurement is the same as described with reference to figure 1 before, except that the temperature at the second place is measured with device 401 instead of device 117 so a repetition of the description may be avoided by refering to figure 1 and the description pertaining to it.

Figure 5 shows the temperature sensor device 401 pierced twice through patient's skin 501. As can be taken from figure 5, the thermal contact portion 404 is in close contact with an artery 407, such as the arteria radialis, femoralis, carotis or any other artery which is close to the skin. As shown in figure 5 the flaps 405 prevent the temperature sensor device 401 from being drawn back. Furthermore, the flaps 405 allow the temperature sensor device 401 to be kept in a well defined position with respect to the pierce hole in the skin 501. An adhesive tape 601 may further secure the temperature sensor device 401 within patient's skin 501 as shown in figure 6.

While the temperature influencing means 107 for provoking an initial local temperature change in the proximity of a first place 101 of a patient's vascular system have been described with respect to the embodiment of figure 1 as a medical dosage device 105 serving together with a catheter 106 as an injection means 107, the temperature influencing means can be replaced with a transcutaneous heat transfer device 701 as illustrated in figure 7.

The heat transfer device 701 as shown in figure 7 is a metallic stick comprising a handle bar 702 and a heat transfer tip 703. The handle bar has an isolating coating 704. For carrying out the thermodilution measurements the heat transfer device 701 is cooled down to a definite temperature and then brought into transcutaneous heat transfer contact with a large vein, such as the vena cava superior or other similar vein, by pressing the tip for a short moment against the said blood vessel. Thereby heat is drawn out of the blood vessel and is absorbed by the heat transfer device 701. As a consequence a travelling temperature deviation is introduced into the patient's vascular system 103 (figure 1) which allows to carry out thermodilution measurements as already described with respect to the embodiment of figure 1.

According to an alternative embodiment of the invention the temperature influencing means 107 may be realized by a subcutaneous extravascular heat transfer device 801 as illustrated in figure 8. The overall structure of the subcutaneous extravascular heat transfer device 801 is very similar to the structure of temperature sensor device 401 as described with reference to figures 4 to 6. However, instead of a thermometer the heat transfer device as illustrated in figure 8 is provided with a Peltier-device, which constitutes a thermal contact portion 804. Also the handling of the subcutaneous extravascular heat transfer device 801 is the same as described with reference to figures 4 to 6, with the only exception that the thermal contact portion 804 of the heat transfer device 801 is brought into contact with a large vein, while the contact portion 804 of the temperature sensor device 801 is brought into contact with a large artery.

According to a further alternative embodiment of the invention in which the temperature influencing means 107 is realized by a subcutaneous extravascular heat transfer device as illustrated in figure 8, instead of a Peltier-device a heating element such as a resistor heater may be provided. In this case the temperature deviation generated in the vein is a rise of temperature instead of a drop of temperature achieved with the Peltier device. The overall structure of the subcutaneous extravascular heat transfer device is equal to the structure as shown in figure 8. Also the handling of the subcutaneous extravascular heat transfer device 801 is the same as described with reference to figure 8, so that repetitions may be avoided.

After having placed the thermal contact portion 804 of the heat transfer device 801 on a large vein, such as the vena cava superior the Peltier-device is energized with the result that its temperature decreases. As the thermal contact portion is in intimate heat transfer contact with the vein, heat is drawn out of the blood vessel and is absorbed by the heat contact portion 804. As a consequence a travelling temperature deviation is introduced into the patient's vascular system 103 (figure 1) which allows to carry out thermodilution measurements as already described with respect to the embodiment of figure 1.

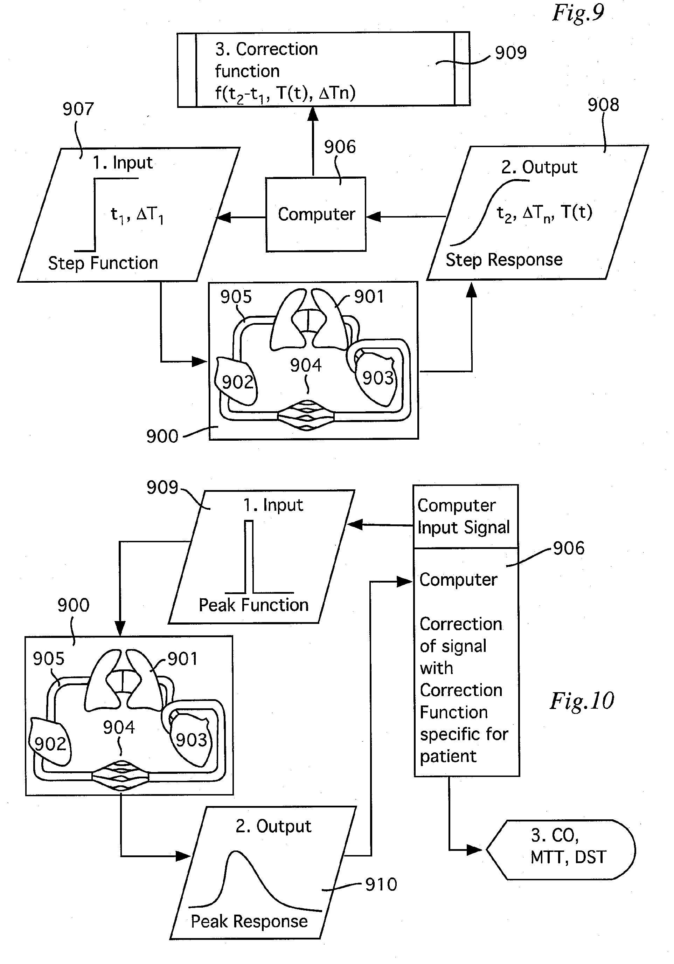

Figure 9 shows a schematic sketch of both a patient's vascular system 900 and a preferred embodiment of computer system 906 adapted to provide data for carrying out a system calibration. Patient's vascular system is generally symbolized by the lungs 901, the right heart ventricle 902, the left heart ventricle 903, the systemic circulation 904 and the pulmonary circulation 905. For better presentation, the right heart ventricle 902 and the left heart ventricle 903 are shown separated from each other.

The computer system 906 including temperature influencing means (not explicitly shown in Fig. 9) induces a change of the blood temperature in a large vein of patient's systemic circulation such that the blood temperature rises by the temperature difference $\Delta T1$ in the form of a step function at time instant t1 as shown in block 907. This causes a temperature rise $\Delta Tn$ at time instant t2 (step response) in an artery of patient's systemic circulation as demonstrated in block 908. This step response curve T(t) is detected by the extravascular temperature sensor device (not explicitly shown in Fig. 9) as a funtion of time and is channelled back to the computer system 906, where it is recorded. The computer then calculates and stores a correction function as shown in block 909 which depends on the time delay (t2 - t1) between the input signal and the output signal and the height of the Step Response $\Delta Tn$.

Fig. 10 shows a schematic sketch of both a patient's vascular system and a preferred embodiment of a computer system adapted carry out a system calibration by means of the correction function provided as explained above with respect to figure 9. As illustrated in figure 10 a temperature change is induced in a large vein of patient's systemic circulation such that the blood temperature rises and falls down to the previous level after a very short time so that the temperature change has the form of a peak function as illustrated in block 909. This causes a temperature rise in an artery of patient's systemic circulation as demonstrated in block 910 (Peak Response). This peak response is detected by the extravascular temperature sensor device, not explicitly shown in Fig. 10) and is channelled back to the computer system 906. The computer then modifies the recorded peak response by means of the correction function which was obtained earlier as demonstrated with respect to figure 9, in order to obtain the thermodilution curve which takes into account the patient's specific parameters such as the heat transfer ability through the skin etc. As usual, parameters such as mean transit time (MTT), downslope time (DST) and cardiac output (CO) can be determined from the thermodilution curve as explained above with respect to figure 3.

**Claims**

1. Apparatus for determining at least one cardio-vascular parameter of a patient by thermodilution measurements comprising:

a) temperature influencing means (107) for provoking an initial local temperature change in the proximity of a first place (101) of a patient's vascular system (103),
b) an extravascular temperature sensor device (117) for measuring the local temperature of patient's blood at a second place (102) of patient's vascular system (103) downstream of said first place (101)
c) a computer system (104) coupled to said temperature sensor device (117) for recording said patient's local blood temperature measured at said second place (102) as a function of time to determine a thermodilution curve (15).

2. Apparatus as claimed in claim 1 wherein said cardiovascular parameter is the cardiac output.

3. Apparatus as claimed in any of the preceding claims wherein said computer system (104) is adapted to determine a downslope of said thermodilution curve (15).

4. Apparatus as claimed in any of the preceding claims wherein said computer system (104) is adapted to determine a mean transit time from said thermodilution curve (15) indicating an estimate of the time required by said temperature deviation to travel from said first place (101) to said second place (102).

5. Apparatus as claimed in any of the preceding claims wherein said extravascular temperature sensor device (117) includes a thermistor.

6. Apparatus as claimed in any of the preceding claims wherein said extravascular temperature sensor device (117) includes a bolometer.

7. Apparatus as claimed in any of the preceding claims wherein the extravascular temperature sensor device (117) is a transcutaneous sensor device which comprises a thermal contact portion adapted to be pressed against the patient's skin to get into heat transfer contact with a blood vessel.

8. Apparatus as claimed in any of claims 1 - 6 wherein the extravascular temperature sensor device (117) is a subcutaneous sensor device which comprises a thermal contact portion adapted to be advanced under the patient's skin to get into heat transfer contact with a blood vessel.

9. Apparatus as claimed in any of the preceding claims wherein the temperature influencing means (107) comprises a thermal contact portion adapted to be pressed against the patient's skin to get into heat transfer contact with a blood vessel.

10. Apparatus as claimed in any of claims 1 - 8 wherein the temperature influencing means (107) comprises a thermal contact portion adapted to be advanced under the patient's skin to get into heat transfer contact with a blood vessel.

11. Computer system (104) for determining at least one cardio-vascular parameter of a patient by thermodilution measurements comprising first coupling means to couple said computer system (104) to temperature influencing means (107) and second coupling means to couple said computer system (104) to an extravascular temperature sensor device (117) and accessing means to acces executable instructions to cause said computer system (104)

a) to control temperature influencing means (107) coupled to said computer system (104) to provoke an initial local temperature change in the proximity of a first place (101) of a patient's vascular system (103), and
b) to record said patient's local blood temperature measured by an extravascular temperature sensor device (117) at a second place (102) of patient's vascular system (103) downstream of said first place (101) as a function of time to determine a thermodilution curve (15).

12. Computer system (104) as claimed in claim 11 wherein said cardiovascular parameter is the cardiac output (CO).

13. Computer system (104) as claimed in claim 11 or 12 wherein said cardiovascular parameter is a downslope time (DST) of said thermodilution curve (15).

14. Computer system (104) as claimed in any of claims 11 to 13 wherein said cardiovascular parameter is a mean transit time (MTT).

15. Computer system (104) as claimed in any of claims 11 to 14 wherein said temperature influencing means (107) is controlled to provoke an initial local temperature change in the form of a step function and to record patient's local blood temperature measured by said extravascular temperature sensor device (117) at said second place (102) of patient's vascular system (103) downstream of said first place (101) as a function of time to determine a step response curve (908), wherein said executable instructions cause said computer system calculate a correction function (909) based on said step response curve (908) and to store said correction function (909) for later calibration purposes.

**16.** Computer system (104) as claimed in claim 15 wherein said thermodilution curve is modified by making use of said correction function (909).

**17.** Computer program for determining a cardio-vascular parameter of a patient by thermodilution measurements comprising instructions executable by a computer system (104) to cause said computer system (104)

a) to control temperature influencing means (107) coupled to said computer system (104) to provoke an initial local temperature change in the proximity of a first place (101) of a patient's vascular system (103), thus introducing a travelling temperature deviation to patient's blood stream,
b) to record said patient's local blood temperature measured by an extravascular temperature sensor device (117) at a second place (102) of patient's vascular system (103) downstream of said first place (101) as a function of time to determine a thermodilution curve (15).

**18.** Computer program as claimed in claim 17 wherein said cardiovascular parameter is the cardiac output.

**19.** Computer program as claimed in claim 17 or 18 wherein said cardiovascular parameter is a downslope of said thermodilution curve (15).

**20.** Computer program as claimed in anyone of claims 17 to 19 wherein said cardiovascular parameter is a mean transit time.

**21.** Computer program as claimed in anyone of claims 17 to 20 wherein said computer program generates a warning signal indicating that cardiac output is below a certain threshold and advising to determine the cardiac output using an invasive temperature sensor device.

**22.** Storage medium (212) having physically stored thereon a computer program as claimed in any of claims 17 - 21.

**23.** A temperature sensor device comprising an elongated body part having a sharp tip (406) at one end,
a temperature sensing section (404) and retaining means (405) adapted to retain the temperature sensor in a fixed place.

**24.** Temperature sensor device as claimed in claim 23, wherein the elongated body part is coated with a foil (403) and the retaining means (405) are flaps obtained by partially peeling off the foil (403).

**25.** Temperature sensor device as claimed in claim 23 or claim 24, wherein the temperature sensing section (404) comprises a thermistor element.

**26.** Temperature sensor device as claimed in anyone of claims 23 to 25, wherein the temperature sensing section (404) comprises a bolometer element.

**27.** A heat transfer device comprising an elongated body part having a sharp tip (406) at one end,
a heat transfer section (404) comprising a heating element or a cooling element, retaining means (405) adapted to retain the heat transfer device in a fixed place.

**28.** Heat transfer device as claimed in claim 27, wherein the heating element is an electric resistance heater.

**29.** Heat transfer device as claimed in claim 27, wherein the cooling element is a Peltier element.

**30.** Heat transfer device as claimed in anyone of claims 27 - 29, wherein the elongated body part is coated with a foil (403) and the retaining means (405) are flaps obtained by partially peeling off the foil (403).

*Fig.1*

*Fig.2*

*Fig.3*

Fig.4

Fig.5

Fig.6

Fig.7

Fig.8

*Fig.9*

3. Correction function
$f(t_2-t_1, T(t), \Delta Tn)$ — 909

907

1. Input

$t_1, \Delta T_1$

Step Function

906

Computer

908

2. Output

$t_2, \Delta T_n, T(t)$

Step Response

905 901
902 904 903
900

*Fig.10*

909 — 1. Input

Peak Function

Computer Input Signal

Computer — 906

Correction of signal with Correction Function specific for patient

900

905 901
902 904 903

2. Output

Peak Response — 910

3. CO, MTT, DST

**European Patent Office** | **EUROPEAN SEARCH REPORT** | Application Number

EP 05 10 8898

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 537 230 B1 (PFEIFFER ULRICH J ET AL) 25 March 2003 (2003-03-25) | 1-4, 7-14, 17-20,22 | A61B5/029 |
| Y | * column 1, line 30 - line 53 * | 5,6,15, 16,21, 25,26,29 | |
| | * column 5, line 42 - column 6, line 21 * <br> * claim 1 * <br> * figures 1,4 * | | |
| Y | US 6 371 923 B1 (ROTELIUK LUCHY D ET AL) 16 April 2002 (2002-04-16) | 5,15,16, 25 | |
| A | * column 1, line 12 * | 1-5,8, 10,13 | |
| | * column 2, line 30 - column 4, line 6 * <br> * column 7, line 40 - column 8, line 22 * <br> * column 12, line 4 - line 24 * <br> * claims 1,7,10,11 * <br> * figures 1,2 * | | |
| X | US 3 542 029 A (MAX L. HIRSCHHORN) 24 November 1970 (1970-11-24) <br> * column 2, line 60 - column 3, line 15 * <br> * figures 1-4 * | 23,27 | TECHNICAL FIELDS SEARCHED (IPC) <br><br> A61B <br> A47J <br> A61M |
| X | US 3 736 859 A (CARLSON A,US) 5 June 1973 (1973-06-05) <br> * column 3, line 32 - column 4, line 25 * <br> * figures 1,3 * | 27,28 | |
| Y | US 2005/094704 A1 (DE CICCO GIORGIO ET AL) 5 May 2005 (2005-05-05) <br> * paragraph [0031] * | 29 | |

-/--

The present search report has been drawn up for all claims

| Place of search | Date of completion of the search | Examiner |
|---|---|---|
| The Hague | 13 March 2006 | Visser, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 05 10 8898

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 6 615 071 B1 (CASSCELLS, III S. WARD ET AL) 2 September 2003 (2003-09-02) | 6,26 | |
| A | * column 1, line 31 - line 32 * <br> * column 4, line 13 - line 57 * <br> * column 5, line 1 - line 10 * <br> * column 18, line 45 - column 19, line 33 * | 1,7,11 | |
| | ----- | | |
| Y | US 5 954 659 A (CURLEY ET AL) 21 September 1999 (1999-09-21) | 21 | |
| A | * column 1, line 10 * | 1,2,5,8, 10,11,15 | |
| | * column 7, line 50 - line 62 * <br> * column 8, line 64 * <br> * column 10, line 52 * <br> * figure 4 * | | |
| | ----- | | |
| A | US 3 526 135 A (EDWARD C. WORTZ) 1 September 1970 (1970-09-01) * column 3, line 60 * | 5,6 | |
| | ----- | | TECHNICAL FIELDS SEARCHED (IPC) |
| X | US 4 633 885 A (DUBRUCQ ET AL) 6 January 1987 (1987-01-06) | 23,25 | |
| A | * column 1, line 1 - line 61 * <br> * column 3, line 65 - column 4, line 30 * <br> * figure 1 * | 1,5 | |
| | ----- | | |

The present search report has been drawn up for all claims

| Place of search | Date of completion of the search | Examiner |
|---|---|---|
| The Hague | 13 March 2006 | Visser, R |

EPO FORM 1503 03.82 (P04C01)

### ANNEX TO THE EUROPEAN SEARCH REPORT
### ON EUROPEAN PATENT APPLICATION NO.

EP 05 10 8898

This annex lists the patent family members relating to the patent documents cited in the above-mentioned European search report.
The members are as contained in the European Patent Office EDP file on
The European Patent Office is in no way liable for these particulars which are merely given for the purpose of information.

13-03-2006

| Patent document cited in search report | | Publication date | Patent family member(s) | | Publication date |
|---|---|---|---|---|---|
| US 6537230 | B1 | 25-03-2003 | NONE | | |
| US 6371923 | B1 | 16-04-2002 | AU | 770070 B2 | 12-02-2004 |
| | | | AU | 2057001 A | 18-06-2001 |
| | | | BR | 0016193 A | 13-08-2002 |
| | | | CA | 2388980 A1 | 14-06-2001 |
| | | | CN | 1409618 A | 09-04-2003 |
| | | | EP | 1235511 A1 | 04-09-2002 |
| | | | JP | 2003515419 T | 07-05-2003 |
| | | | WO | 0141635 A1 | 14-06-2001 |
| US 3542029 | A | 24-11-1970 | NONE | | |
| US 3736859 | A | 05-06-1973 | NONE | | |
| US 2005094704 | A1 | 05-05-2005 | AU | 2002367115 A1 | 15-07-2003 |
| | | | CA | 2470874 A1 | 10-07-2003 |
| | | | EP | 1458434 A1 | 22-09-2004 |
| | | | WO | 03055544 A1 | 10-07-2003 |
| | | | IT | MI20012828 A1 | 30-06-2003 |
| | | | JP | 2005512737 T | 12-05-2005 |
| US 6615071 | B1 | 02-09-2003 | NONE | | |
| US 5954659 | A | 21-09-1999 | NONE | | |
| US 3526135 | A | 01-09-1970 | NONE | | |
| US 4633885 | A | 06-01-1987 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9321823 A
- US 20030130587 A1